# EUROPEAN PATENT SPECIFICATION

(11) **EP 3 778 024 B1**
(45) Date of publication and mention of the grant of the patent: **03.08.2022**
(21) Application number: 19192141.0
(22) Date of filing: 16.08.2019
(51) Int. Cl.: B01L 3/00, G01N 33/543, G01N 21/552, G01N 21/65

(54) **DEVICE AND METHOD FOR SIZE-SELECTIVE PARTICLE SEPARATION, TRAPPING, AND MANIPULATION OF MICRO AND NANOPARTICLES FOR MOLECULAR DETECTION**
VORRICHTUNG UND VERFAHREN ZUR GRÖSSENSELEKTIVEN PARTIKELABSCHEIDUNG, EINFANGEN UND MANIPULATION VON MIKRO- UND NANOPARTIKELN ZUR MOLEKULAREN DETEKTION
DISPOSITIF ET PROCÉDÉ DE SÉPARATION DE PARTICULES SELON LA TAILLE, DE PIÉGEAGE ET DE MANIPULATION DE MICRO ET NANOPARTICULES POUR LA DETECTION MOLÉCULAIRE

(43) Date of publication of application: 17.02.2021
(73) Proprietor: PAUL SCHERRER INSTITUT, 5232 Villigen PSI (CH)
(72) Inventor: EKINCI, Yasin, 8046 Zürich (CH); SHARMA, Deepika, 4056 Basel (CH)
(74) Representative: Fischer, Michael

(56) References cited:
- US-A1- 2009 214 392
- US-A1- 2014 252 505
- US-A1- 2016 320 389
- US-A1- 2018 305 682
- BENJAMIN H. WUNSCH ET AL: "Nanoscale lateral displacement arrays for the separation of exosomes and colloids down to 20?nm", NATURE NANOTECHNOLOGY, vol. 11, no. 11, 1 August 2016 (2016-08-01), pages 936-940, XP055629143, London ISSN: 1748-3387, DOI: 10.1038/nnano.2016.134

## Description

The present invention relates to a device and a method for size-selective particle separation, trapping, and manipulation of micro and nanoparticles for molecular detection. Microfluidics has been an emerging and promising area of research and development for both applied and basic research in the fields of biology, fluid dynamics, material science, and diagnostics. Integrated micro/nanofluidic devices furthermore use both surface-specific properties of nanostructures as well as controlled fluid dynamics of microstructures to provide a platform for molecular dynamic, intermolecular interactions studies, and molecular sensing for nanoscale molecules at low concentration. In various applications micro- and nanofluidic devices have been used for molecule separation, sorting and trapping.

Document US 2016/320389 A1 discloses a virus detection chip device comprising a capillary opening, a first filtering nanopillar array, a second nanopillar array, connected to the first nanopillar array, and a diode-induced fluorescence detector, connected to the second nanopillar array. Document US 2014/252505 A1 discloses an analysis microchip comprising a columnar structure array with a plurality of nanopillars. Document US 2009/214392 A1 discloses a nano-fluidic trapping device for assembling a SERS-active cluster comprising a SERS-active cluster compartment having a plurality of nanopillars.

Integrated micro/nanofluidic solutions for Lab-on-α-chip (LoC) platforms for diagnostic applications have been of great interest in both diagnostic as well as in preventive detection of diseases. Most of the existing portable diagnostic solutions either uses surface functionalization of LoC device to capture the target molecule to perform qualitative detection or perform quantitative detection using SERS substrates (SERS = Surface-Enhanced Raman Spectroscopy).

However, due to the shortcomings of surface functionalization, these solutions are not effective for multi-disease diagnosis on a single chip in a cost-effective manner with both qualitative and quantitative information. Many of the existing solutions also require sample preparation before performing the detection, thus making it difficult to be used by untrained medical staff members or patient itself.

Thus, it is the objective of the present invention to enable molecular detection at very small target molecule concentrations (a few pg/ml) in presence of whole blood, urine, etc. through a robust method which can provide conclusive quantitative information of multiple biomarkers on a single chip.

This objective is achieved according to the present invention by a lab-on-α-chip (LoC) device for size-selective separation, trapping, and manipulation of micro/nanoparticles from a flow of a sample solution for molecular detection, comprising:
a) an in-port area for the reception of a sample solution;
b) a micro separation area and a macro particle separation area, preferably designed as common macro- and micro-particle separation area;
c) a micro/nanoparticles sorting and separation area;
d) a functionalized nanoparticle trapping and detection area; and
e) an out-port area for the waste sample solution.

Therefore, the present device represents a LoC platform that allows for particle separation, sorting and trapping at specific locations in the device for molecular sensing in the range of nano- to picomolar concentration. In combination with optical detection using surface-enhanced Raman spectroscopy (SERS) and localized surface plasmon resonance (LSPR), a highly sensitive (~10 pg/ml) molecular detection can be achieved.

Practically, the in-port area may be designed as a blood drop injection area.

With respect to the claimed invention, the following features are also used:
a) the in-port area (4) comprises a micro pillar array (14);
   said micro pillar array (14) is patterned with pillars having
   a diameter ranging from 1 to 50 µm, preferably ranging around 15 µm, with and inter-pillar spacing ranging from 1 to 50 µm, preferably ranging around 15 µm;
b) the macro particle separation area (6) and the micro particle
   separation area (8) comprise a pillar array having pillars with a diameter in the range from 1 to 20 µm, preferably ranging around 3 µm, with an inter-pillar spacing in the range from 0.5 to 20 µm, preferably ranging around 2 to 5 µm, being connected with an area patterned with rectangular pillars in the range of 1 to 20 µm x 1 to 20 µm, preferably ranging around 5 µm x 3 µm, being rotated at an angle in the range of 30 to 70°, preferably ranging from 45 to 55°, with an interpillar spacing in the range of 1 to 10 µm in axial and lateral direction of the flow of the sample solution, preferably around 6 µm and 6.6 µm inter-pillar spacing in axial and lateral direction of the flow of the sample solution;
c) the nanoparticles sorting and separation area (10) comprises nanochannels embedded with a deterministic lateral displacement (DLD) array; said DLD array is achieved using 0.5 to 10 µm, preferably 2 µm, diameter pillars with 0.2 to 5 µm, preferably 1 µm and 500 nm, inter-pillar spacing. The following feature may also be used:
d) the DLD arrays is achieved for period (N) = 1, 2, 3, 10, 20 with 1 µm inter-pillar spacing and for period N = 1, 10, 12, 18, 25 with 500 nm inter-pillar spacing.

Furthermore, in the context of the claimed invention, in order to trap particles having different sizes, the functionalized nanoparticle trapping and detection area (12) comprises a number of stages or steps (16a to 16c) that are patterned with different height to trap different particles (18a to 18c) at different stage location based on the particle size; said stages or steps (16a to 16c) preferably are supported with pillars to avoid pattern collapsing.

Further, the objective mentioned above is achieved according to the present invention by a method for size-selective particle separation, trapping, and manipulation of micro/nanoparticles from a flow of a sample solution for molecular detection; comprising the steps of:
a) providing a sample solution to be investigated;
b) conveying - substantially capillary force-driven- the sample solution through a LoC device according to any of the claims 1 to 3.

Further, prior to introducing the sample solution, a solution comprising functionalized nanoparticles may be conveyed through the LoC device; said functionalization reflects the biomolecules intended to be trapped in the functionalized nanoparticle trapping and detection area of said LoC device. Alternatively or additionally, a solution comprising functionalized nanoparticles may be added to the sample solution before the sample solution is conveyed through the LoC device; said functionalization reflects the biomolecules intended to be trapped in the functionalized nanoparticle trapping and detection area of said LoC device.

In order to further investigate the biomolecules the LoC device was used to detect for, the biomolecule load trapped in the functionalized nanoparticle trapping at the detection area of said LoC device can be investigated using optical detection to acquire surface-enhanced Raman spectroscopy (SERS) signal of the antibody-antigen complex or fluorescent signal from the molecular assembly attached on the surface of these particles.

Preferred embodiments of the present invention are hereinafter described in more detail with respect to the attached drawings which depict in:
- Fig. 1: schematically shows the architecture of lab-on-a-chip platform device (top view) for early disease detection using antibody-functionalized nanoparticles;
- Fig. 2: schematically nanochannels embedded with deterministic lateral displacement arrays and three steps for passive size-based trapping of three different nanoparticles within the device according to Fig. 1;
- Fig. 3: schematically the architecture and the substantial production steps of the lab-on-α-chip (LoC) platform device according to Fig. 1 using state-of-the-art lithography processes and replica molding using polydimethylsiloxane (PDMS); and
- Fig. 4: schematically a microarray of 1 µm diameter pillars with 1 µm inter-pillar spacing inside the integrated micro/nanofluidic LoC device according to Fig. 1 for size-based sorting of nanoparticle and directional motion of the particles of interest.

The present invention discloses a device 2 that incorporates the field of micro- and nano-fluidics for particle separation, trapping, and manipulation in different particle size ranges (from microscale to nanoscale). The device 2 is specifically designed to use whole blood sample as well as other fluidic solutions for the detection of target biomolecules that could be disease-specific antigens, lipids, proteins, virus or other biomolecules present in the solution. To perform the molecular detection the device 2 uses size-based particle separation and trapping of the micro/nanoparticles that are functionalized with the antibodies to capture the target molecules from the solution. Trapped nanoparticles are further used for optical detection to acquire surface-enhanced Raman spectroscopy (SERS) signal of the antibody-antigen complex or fluorescent signal from the molecular assembly attached on the surface of these particles.

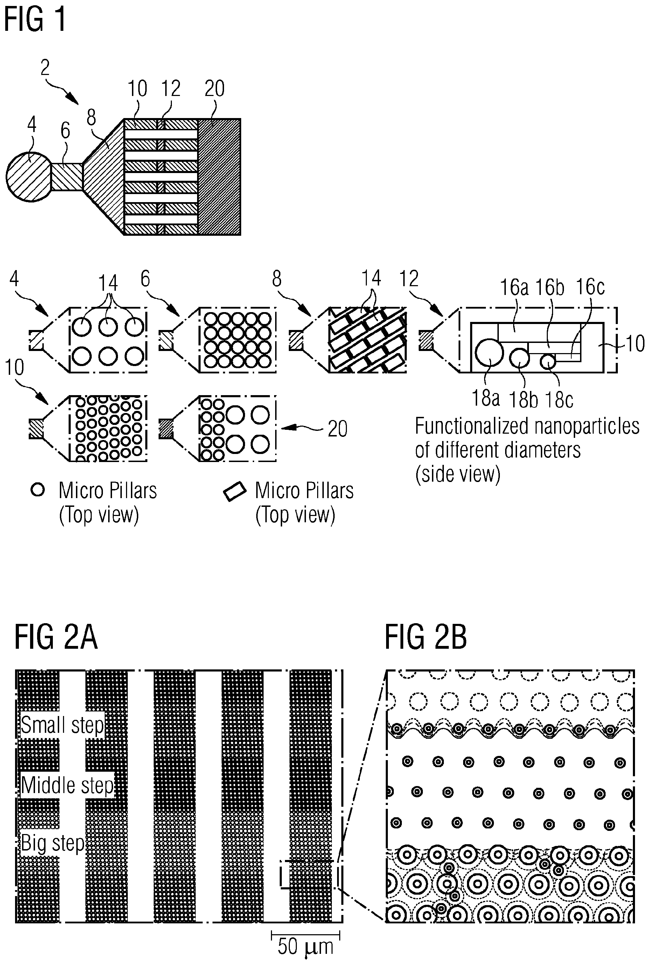

Figure 1 schematically shows the architecture of lab-on-α-chip platform device 2 (top view) for early disease detection using antibody-functionalized nanoparticles. A blood sample is injected at an in-port area 4 in combination with functionalized nanoparticles allowing antibody-antigen binding on a nanoparticle surface, and detection of bound antibodies at a detection area. The blood sample solution is conveyed by capillary forces through the different sections of the LoC device 2. The in-port (injection) area 4, a macro particle separation area 6 and a micro particle separation area 8, a nanoparticle separation area 10, all comprise micro array pillars 14 with different inter-pillar spacing, pillar period and orientation. A functionalized nanoparticle trapping and detection area 12 comprises three different stages 16a to 16c to allow for size-based trapping of three different diameter nanoparticles 18a to 18c at different locations within this detection area 12.

The LoC device 2 is specifically designed to use functionalized nanoparticles for early disease diagnosis, where nanoparticles can be functionalized with disease-specific antibodies. The device 2 has one in-port area 4 and one out-port area 20 as shown in Figure 1. Both in-port area 4 and out-port area 20 are patterned with circular pillars 14 to support microstructures and avoid pattern collapsing after transferring the device pattern to elastic materials such as polydimethylsilaxone (PDMS). The sample solution is conveyed by capillary forces from the in-port area 4 to the out-port area 20 thereby passing all components being arranged in between.

The out-port area 20 is patterned with 3 µm and 15µm diameter circular pillars with 7µm and 15 µm inter-pillar spacing, respectively, whereas the in-port area 4 is patterned with 15 µm diameter pillars with 15 µm inter-pillar spacing. For macro- and micro-particle separation from the sample solutions, 3 µm pillars are patterned with 2 µm inter-pillar spacing and connected with an area patterned with 5 µm x 3 µm rectangular pillars rotated at an angle of ≈ 48.37° with 6 µm and 6.6 µm inter-pillar spacing in axial and lateral direction of the flow of the sample solution, respectively. The macro/nanoparticle separation area 6, 8 is directly connected with nanochannels embedded with deterministic lateral displacement (DLD) array. DLD arrays were achieved using 2 µm and 1µm diameter pillars with 1 µm and 500 nm inter-pillar spacing. The DLD arrays were achieved for a period (N) = 1, 2, 3, 10, 20 with 1 µm inter-pillar spacing and for N = 1, 10, 12, 18, 25 with 500 nm inter-pillar spacing. Patterned DLD arrays allow for nanoparticle sorting and separation. It furthermore promotes the directional motion of the nanoparticles helping them to be trapped at a specific direction/location. In the particle detection area, stages are patterned with different heights to trap different particles at different stage location based on the particle size as shown in Fig. 1 and Fig. 2. The stages or steps are supported with pillars to avoid pattern collapsing as shown in Fig. 2 which depicts schematically nanochannels embedded with deterministic lateral displacement arrays and three steps for passive size-based trapping of three different nanoparticles. Fig. 2(b) shows the trapping of 150 nm diameter gold nanoparticles at the location of a big step inside the nanochannel; Fig. 2(a) shows nanochannels being imbedded with 2 µm diameter DLD pillars which also serve as supporting pillars for the nanosteps and nanochannels.

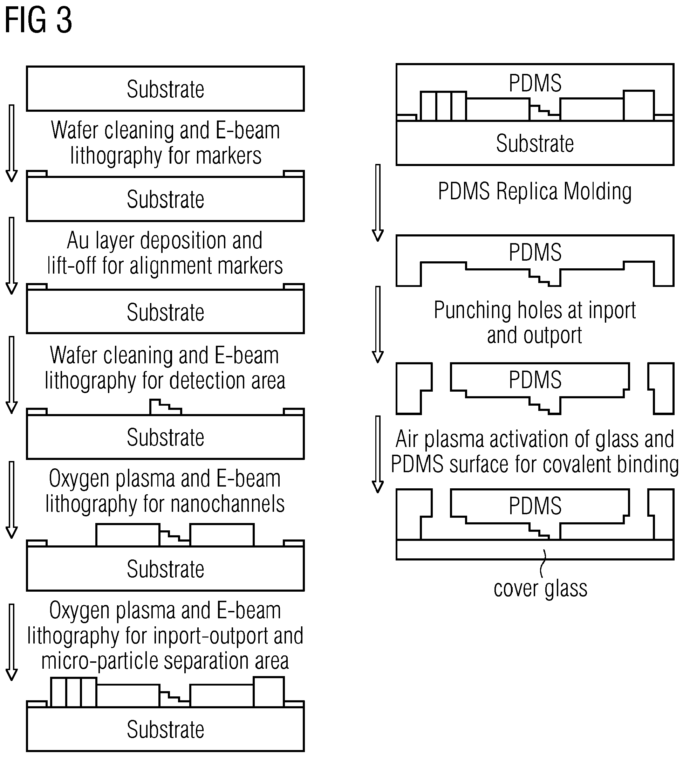

The lab-on-α-chip (LoC) platform device (shortly also referred to as LoC device) for biosensing applications is developed using state-of-the-art lithography processes and replica molding using polydimethylsiloxane (PDMS)which is schematically shown in Figure 3. The final integrated micro/nanofluidic device was developed using air plasma activation of patterned PDMS and a cover glass.

In detail, a negative master was developed on a Si wafer using electron-beam lithography (Vistec EBPG 5000 Plus). As shown in Figure 3, a silicon wafer was sonicated under acetone and isopropyl alcohol (IPA) for 10 min in each solvent, and dried under nitrogen (N2) gas stream. The cleaned wafer was further processed for 1 min in buffered hydrofluoric acid (BOE 7:1, HF:NH4F = 12.5:87.5%, General Chemical), and later washed thoroughly with Deionized (DI) water and N2 dried. To proceed with the alignment marker patterning, the wafer was spin-coated (1000 rpm, 300 rpm/s, 60 s) with poly-(methylmethacrylate) (PMMA, 950K, 4% ethylacetate, Allresist), and pre-baked at 175 °C for 4 min.

After the e-beam exposure of alignment markers pattern, PMMA was developed under methyl-isobutyl-ketone (MIBK):IPA (1:1) for 60 s. A 60 nm thick gold (Au) layer was thermally deposited on the patterned wafer after the deposition of a 5 nm thin interconnect layer of chromium, transferring the patterns on the wafer. The PMMA resist was removed from the wafer through Acetone, IPA, DI sonication, and cleaned wafer was processed with 02 plasma.

Immediately after plasma cleaning, HSQ Fox16 (1:4) negative e-beam resist was spin coated on the wafer (3000 rpm, 500 rpm/s, 60 s). The E-beam exposure for the small steps was done and after the exposure the resist was developed in AZ351:H2O = 1:3 for 3 min. The wafer was further cleaned using 02 plasma for 2 min, and HSQ Fox16 (1:3) resist was spin coated (4000 rpm, 500 rpm/s, 60 s).

The pattern for the middle step was exposed using e-beam, and the resist was developed in AZ351:H2O = 1:3 solution for 3 min. For big step patterning, the wafer was 02 plasma cleaned for 2 min and spin-coated with HSQ Fox16 (1:2) (1500 rpm, 500 rpm/s, 60 s) before the e-beam exposure. The exposed resist was developed for 3 min, and the wafer was 02 plasma cleaned for 2 min. The cleaned wafer was spin coated with HSQ Fox (1:1) resist (1000 rpm, 500 rpm/s, 60 s), and exposed with the pattern for the nanochannels. After the development of the exposed resist for 3 min, the wafer was again cleaned with 02 plasma, and spin coated with HSQ Fox (undiluted) resist (1000 rpm/s, 300 rpm, 60 s). The wafer was exposed with in-port and out-port areas, and developed in the developer solution for 7 min and later sonicated in the developer solution for 6 min. the final thickness of small, middle, and big steps/stages are approximately 78 nm, 100 nm, 170 nm, respectively. The thickness of the nanochannels and the in/out-port areas are ~330 nm and ~885 nm, respectively.

The device pattern was transferred to PDMS mold using replica molding as shown in Figure 3, after performing gas phase silanization of the patterned silicon wafer. Silanization was performed inside a vacuum chamber using a mixture of 1:1 trichloro(1H,1H,2H,@H-perfluorooctyl) silane and (tridecafluoro-1,1,2,2-tetrahydrooctyl)dimethylchlorosilane for 10 min. For replica molding UV curable PDMS base and curing agent mixture (ShinEtsu Chemical Co. Ltd.) was used in 1:1 weight ratio and cured for 4 min under UV exposure at 30mW/cm2 and then baked at 50°C in an oven at atmospheric pressure. The patterned PDMS mold was further prepared for final device assembly by punching holes at the in/out-port locations. Immediately before the final device assembly a cleaned cover glass and a prepared PDMS mold were processed with air plasma for the activation of glass and PDMS surface for effective covalent binding. Before pressing both the surfaces together, the nanostructures of the patterned PDMS mold were filled with 2.5 µl of DI water through capillary force reaction. The pre-filling of the nanostructures in the patterned PDMS surface avoids a pattern collapsing during the glass-PDMS surface binding process.

The device was specifically designed to use functionalized nanoparticles for early disease diagnosis, where nanoparticles can be functionalized with disease-specific antibodies. Antibody functionalized nanoparticles can be purchased directly or prepared in the laboratory using surface chemistry. To perform experiments for disease-specific biomarker detection, nanoparticles need to be mixed with body fluid and injected through the in-port area into the LoC device. The LoC platform device comprises areas specifically designed for passive separation of micro molecules from the sample solution and size-based trapping of nanoparticles at the detection area, enabling multi-particle detection on a single device as shown in Fig. 1.

In combination with the gold nanoparticles functionalized with antibodies related to the biomolecule of interest, the LoC device provides a means to detect biomarkers using surface-enhanced Raman spectroscopy (SERS).

By performing the optical measurement on trapped nanoparticles one can get both qualitative and quantitative information of the disease specific biomarkers. Here, Raman measurements on trapped gold nanoparticles were performed at the detection area of LoC device.

Microparticle separation experiments were performed using whole blood sample mixed with gold nanoparticles. For detection of disease-specific biomolecule detection, it is important to separate red and white blood cells, and platelets including other micro and macro particles from the gold nanoparticles at the detection area to achieve high signal to noise ratio of SERS signal from the attached molecules on the functionalized nanoparticles. Thus, preliminary experiments were performed to identify the efficiency of the device to separate these particles before the nanochannel region in the LoC device. Based on the performed experiments, it was identified that for a mixture of 100 µl of the whole blood sample and 10 µl of (-ve) 80 nm of AuNPs, all macro and micro molecules sieved out before the nanochannel region.

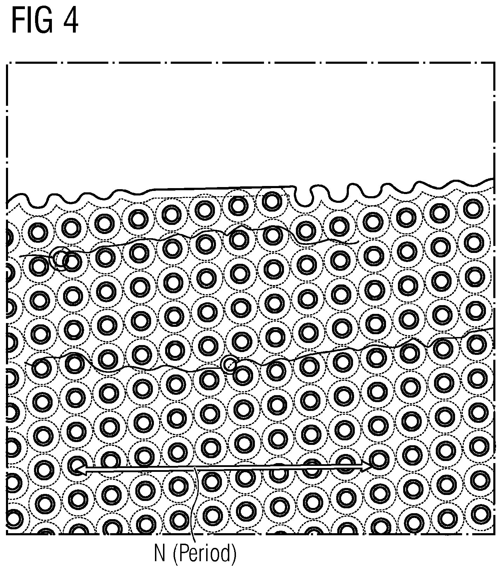

Passive sorting of nanoparticles was achieved by deterministic lateral displacement (DLD) using different micro-structured arrays integrated on the LoC device as shown in Fig. 4. Figure 1 exemplarily shows a microarray of 1 µm diameter pillars with 1 µm inter-pillar spacing inside the integrated micro/nanofluidic device for size-based sorting of nanoparticle and directional motion of the particles of interest. The presence of deterministic lateral displacement arrays with a period (N) = 10 assisted the directional motion of nanoparticles with a diameter of 200 nm.

Particularly, the DLD arrays support both size-based particle separation as well as the directional displacement of the nanoparticles. The size-based nanoparticle sorting and the directional motion was investigated using DLD arrays with 1 µm diameter pillars with 2 µm and 1 µm inter-pillar spacing in the both axial and lateral directions of the flow. The DLD arrays were patterned with an array period (N) of 0, 1, 2, 10, 100, 200, 300, and 600. Preliminary experiments were performed using 150 nm and 200 nm Au NPs to examine the efficiency of the DLD arrays to allow motion of nanoparticle in the direction of the pillar array. During the experiments, it was observed that among all DLD arrays, the array with 1 µm inter-pillar spacing and an array period of N = 10 promoted the directional motion of both 200 nm and 150 nm Au NPs as shown in Fig. 4.

However, at the aspect ratio (height : width) 1000 : 330, the 1 µm diameter pillars inside the nanochannels were collapsing during the experiments due to nanochannel drying. Furthermore, for an array period N ≥ 100, the lateral row shift was not sufficient enough to allow for the directed motion of the nanoparticles along the array direction.

Thus, the next generation of the LoC device design was developed using DLD arrays with periods lower than 100 and an inter-pillar spacing of 1 µm and 500 nm for a 2 µm diameter circular array as mentioned above. The reduced aspect ratio of the pillars inside the nanochannel region contributed to the stability of the pillars. However, the directional motion of nanoparticles for new set of DLD arrays was not as effective as observed for 1 µm diameter pillars with inter-pillar spacing of 1 µm. Further optimization of the DLD array will lead to location specific trapping of nanoparticles based on their sizes in the detection area as shown in Figure 4, which would allow multiplexable detection on a single LoC platform device.

The detection area in the LoC platform device was patterned with nanometric steps/stages as shown in Figure 3. The step structures allow for passive size-based trapping of nanoparticles, leading to the desired possibility of multi-diametric particle detection at the detection area.

Several experiments with two and three steps for 2 and 3 different diametric particles trapping at the detection area have been performed. The achievement was that the trapping of nanoparticles (250 nm to 100 nm) at the first step and the removal of (~ 60 nm) smaller particles through the opening had been established successfully. For the separation of 200 nm and 100 nm nanoparticle at the detection area, further optimization of the step heights can be chosen according to the effective needs and results to be achieved.

Preliminary sensing experiments were performed in different buffer solutions using 80 nm diameter gold nanoparticles which comprise -COOH groups on the particle surface. The presence of gold nanoparticles enhances the electric field coupled with the particles leading to both SERS signal as well as nanoparticle agglomeration and melting due to overheating and plasmon coupling. Thus, both detection settings and methods have to be adjusted accordingly for the precise detection of SERS signal from the molecules attached on the nanoparticle surface. Nonetheless, the present LoC platform device allows for multi-biomarker detection on a single chip.

## Claims

1. A lab-on-α-chip (LoC) device (2) for size-selective particle separation, trapping, and manipulation of micro/nanoparticles from a flow of a sample solution for molecular detection, comprising:
a) an in-port area (4) for the reception of a sample solution wherein the in-port area (4) comprises a micro pillar array (14) being patterned with pillars having a diameter ranging from 1 to 50 µm, preferably ranging around 15 µm, with an inter-pillar spacing ranging from 1 to 50 µm, preferably ranging around 15 µm;
b) a macroparticle separation area (6) and a microparticle separation area (8) disposed downstream of the macroparticle separation area (6) wherein the microparticle separation area (8) and the macroparticle separation area (6) comprise a pillar array (6) having pillars with a diameter in the range from 1 to 20 µm, preferably ranging around 3 µm, with an inter-pillar spacing in the range from 0.5 to 20 µm, preferably ranging around 2 to 5 µm, being connected with an area (8) patterned with rectangular pillars in the range of 1 to 20 µm x 1 to 20 µm, preferably ranging around 5 µm x 3 µm, the rectangular pillars being rotated at an angle in the range of 30 to 70°, preferably ranging from 45 to 55°, with an interpillar spacing in the range of 1 to 10 µm in axial and lateral direction of the flow of the sample solution, preferably around 6 µm and 6.6 µm inter-pillar spacing in axial and lateral direction of the flow of the sample solution;
c) a nanoparticle sorting and separation area (10) being disposed downstream of the microparticle separation area (8) and comprising nanochannels embedded with a deterministic lateral displacement (DLD) array; said DLD array is achieved using 0.5 to 10 µm, preferably 2 µm and 1 µm, diameter pillars with 0.2 to 5 µm, preferably 1 µm and 500 nm, inter-pillar spacing;
d) a functionalized nanoparticle trapping and detection area (12) being disposed in the nanoparticle sorting and separation area (10) and comprising a number of stages or steps (16a to 16c) that are patterned with different heights to trap different particles (18a to 18c) at different stage locations based on the particle size; said stages or steps (16a to 16c) preferably are supported with pillars to avoid pattern collapsing; and
e) an out-port area (20) for the waste sample solution.

2. The LoC device (2) according to claim 1 wherein the in-port area (4) is designed as blood drop injection area.

3. The LoC device (2) according to claim 1, wherein the DLD arrays is achieved for period N = 1, 2, 3, 10, 20 with 1 µm inter-pillar spacing or for period N = 1, 10, 12, 18, 25 with 500 nm inter-pillar spacing.

4. A method for size-selective particle separation, trapping, and manipulation of micro/nanoparticles from a flow of a sample solution for molecular detection; comprising the steps of:
a) providing a sample solution to be investigated;
b) conveying - substantially capillary-force driven - the sample solution through a LoC device (2) according to any of the preceding claims 1 to 3.

5. The method according to claim 4 wherein before introducing the sample solution, a solution comprising functionalized nano particles is conveyed through the LoC device (2); said functionalization reflects the biomolecules intended to be trapped in the functionalized nanoparticle trapping and detection area (12) of said LoC device (2).

6. The method according to claim 4 wherein a solution
comprising functionalized nano particles is added to the sample solution before the sample solution is conveyed through the LoC device (2); said functionalization reflects the biomolecules intended to be trapped in the functionalized nanoparticle trapping and detection area (12) of said LoC device (2).

7. The method according to claim 5 or 6, wherein a biomolecule load trapped in the functionalized nanoparticle trapping and detection area (12) of said LoC device (2) is investigated using optical detection to acquire SERS (surface enhanced Raman spectroscopy) signal of the antibody-antigen complex or fluorescent signal from the molecular assembly attached on the surface of these particles.

## Patentansprüche

1. Lab-on-a-Chip (LoC)-Vorrichtung (2) zur größenselektiven Partikelabscheidung, Einfangen und Manipulation von Mikro-/ Nanopartikeln aus einer Probenlösung zur molekularen Detektion, die Folgendes umfasst:
a) einen Einlassbereich (4) für die Aufnahme einer Probenlösung, wobei der Einlassbereich (4) eine Mikrosäulen-Anordnung (14) umfasst, die mit Säulen strukturiert ist, die einen Durchmesser von 1 bis 50 µm, vorzugsweise von etwa 15 µm, mit einem Abstand zwischen den Säulen von 1 bis 50 µm, vorzugsweise von etwa 15 µm, aufweisen,
b) einen Makropartikel-Abscheidebereich (6) und einen Mikropartikel-Abscheidebereich (8), der stromabwärts des Makropartikel-Abscheidebereichs (6) angeordnet ist, wobei der Makropartikel-Abscheidebereich (6) und der Mikropartikel-Abscheidebereich (8) eine Säulen-Anordnung (6) umfasst, die Säulen mit einem Durchmesser im Bereich von 1 bis 20 µm, vorzugsweise von etwa 3 µm, mit einem Abstand zwischen den Säulen im Bereich von 0,5 bis 20 µm, vorzugsweise von etwa 2 bis 5 µm, aufweist, die mit einem Bereich (8) verbunden sind, der mit rechteckigen Säulen im Bereich von 1 bis 20 µm x 1 bis 20 µm, vorzugsweise von etwa 5 µm x 3 µm, strukturiert ist, wobei die rechteckigen Säulen in einem Winkel im Bereich von 30 bis 70°, vorzugsweise von etwa 45 bis 55°, gedreht sind, mit einem Abstand zwischen den Säulen im Bereich von 1 bis 10 µm in axialer und lateraler Richtung des Flusses der Probenlösung, vorzugsweise etwa 6 µm und 6,6 µm Abstand zwischen den Säulen in axialer und lateraler Richtung des Flusses der Probenlösung,
c) einen Nanopartikel-Sortier- und Abscheidebereich (10), der stromabwärts des Mikropartikel-Abscheidebereichs (8) angeordnet ist und Nanokanäle umfasst, die in eine DLD-Anordnung (Deterministic Lateral Displacement, deterministische laterale Verschiebung) eingebettet sind, wobei die DLD-Anordnung unter Verwendung von Säulen mit einem Durchmesser von 0,5 bis 10 µm, vorzugsweise 2 µm und 1 µm, mit einem Abstand zwischen den Säulen von 0,2 bis 5 µm, vorzugsweise 1 µm und 500 nm, erreicht wird,
d) einen funktionalisierten Nanopartikel-Einfang- und Detektionsbereich (12), der in dem Nanopartikel-Sortier- und Abscheidebereich (10) angeordnet ist und eine Anzahl von Stufen oder Versätzen (16a bis 16c) umfasst, die mit unterschiedlichen Höhen strukturiert sind, um unterschiedliche Partikel (18a bis 18c) an unterschiedlichen Stufenpositionen auf Grundlage der Partikelgröße einzufangen, wobei die Stufen oder Versätze (16a bis 16c) vorzugsweise mit Säulen gestützt werden, um ein Zusammenbrechen der Struktur zu vermeiden.
e) einen Auslassbereich (20) für die Abfall-Probenlösung.

2. LoC-Vorrichtung (2) nach Anspruch 1, wobei der Einlassbereich (4) als Bereich zur Einspritzung von Blutstropfen ausgebildet ist.

3. LoC-Vorrichtung (2) nach Anspruch 1, wobei die DLD-Anordnung für Periode N = 1, 2, 3, 10, 20 mit 1 µm Abstand zwischen den Säulen oder für Periode N = 1, 10, 12, 18, 25 mit 500 nm Abstand zwischen den Säulen erreicht wird.

4. Verfahren zur größenselektiven Partikelabscheidung, Einfangen und Manipulation von Mikro-/Nanopartikeln aus einem Fluss einer Probenlösung zur molekularen Detektion, das die folgenden Schritte umfasst:
a) Bereitstellen einer zu untersuchenden Probenlösung,
b) Befördern - im Wesentlichen kapillarkraftgetrieben - der Probenlösung durch eine LoC-Vorrichtung (2) nach einem der vorhergehenden Ansprüche 1 bis 3.

5. Verfahren nach Anspruch 4, wobei vor Einführung der Probenlösung eine Lösung, die funktionalisierte Nanopartikel umfasst, durch die LoC-Vorrichtung (2) befördert wird, wobei die Funktionalisierung die Biomoleküle, die bestimmt sind, in dem funktionalisierten Nanopartikel-Einfang- und Detektionsbereich (12) der LoC-Vorrichtung (2) eingefangen zu werden, nachempfindet.

6. Verfahren nach Anspruch 4, wobei eine Lösung, die funktionalisierte Nanopartikel umfasst, zu der Probenlösung hinzugefügt wird, bevor die Probenlösung durch die LoC-Vorrichtung (2) befördert wird, wobei die Funktionalisierung die Biomoleküle, die bestimmt sind, in dem funktionalisierten Nanopartikel-Einfang- und Detektionsbereich (12) der LoC-Vorrichtung (2) eingefangen zu werden, nachempfindet.

7. Verfahren nach Anspruch 5 oder 6, wobei eine in dem funktionalisierten Nanopartikel-Einfang- und -Detektionsbereich (12) der LoC-Vorrichtung (2) eingefangene Biomolekülbeladung mittels optischer Detektion untersucht wird, um das SERS-Signal (Surface Enhanced Raman Spectroscopy, oberflächenverstärkte Raman-Spektroskopie) des Antikörper-Antigen-Komplexes oder das Fluoreszenzsignal von der molekularen Anordnung, die an die Oberfläche dieser Partikel binden, zu erfassen.

## Revendications

1. Dispositif (2) de laboratoire-sur-puce (LsP) pour la séparation de particules selon la taille, le piégeage, et la manipulation de micro / nanoparticules dans un flux d'une solution d'échantillon pour la détection moléculaire, comprenant :
a) une zone d'entrée (4) pour la réception d'une solution d'échantillon dans laquelle la zone d'entrée (4) comprend un réseau de micro-piliers (14) étant structuré avec des piliers ayant un diamètre de l'ordre de 1 à 50 µm, de préférence de l'ordre de 15 µm environ, avec un espacement entre piliers de l'ordre de 1 à 50 µm, de préférence de l'ordre de 15 µm environ ;
b) une zone de séparation des macroparticules (6) et une zone de séparation des microparticules (8) disposée en aval de la zone de séparation des macro-particules (6), la zone de séparation des microparticules (8) et la zone de séparation des macroparticules (6) comprenant un réseau de piliers (6) ayant des piliers avec un diamètre de l'ordre de 1 à 20 µm, de préférence de l'ordre de 3 µm environ, avec un espacement entre piliers de l'ordre de 0,5 à 20 µm, de préférence de l'ordre de 2 à 5 µm environ, étant raccordée à une zone (8) structurée avec des piliers rectangulaires de l'ordre de 1 à 20 µm x 1 à 20 µm, de préférence de l'ordre de 5 µm x 3 µm environ, les piliers rectangulaires étant tournés selon un angle de l'ordre de 30 à 70°, de préférence de l'ordre de 45 à 55°, avec un espacement entre piliers de l'ordre de 1 à 10 µm dans un sens axial et latéral du flux de la solution d'échantillon, de préférence avec un espacement entre piliers de 6 µm et 6,6 µm environ dans le sens axial et latéral du flux de la solution d'échantillon ;
c) une zone de tri et de séparation des nanoparticules (10) étant disposée en aval de la zone de séparation des microparticules (8) et comprenant des nanocanaux intégrés avec un réseau de déplacement latéral déterministe (DLD) ; ledit réseau DLD est réalisé en utilisant des piliers avec un diamètre de 0,5 à 10 µm, de préférence, 2 µm et 1 µm, avec un espacement entre piliers de 0,2 à 5 µm, de préférence, de 1 µm et 500 nm ;
d) une zone de piégeage et de détection de nanoparticules fonctionnalisées (12) étant disposée dans la zone de triage et de séparation des nanoparticules (10) et comprenant un certain nombre de niveaux ou d'étapes (16a à 16c) qui sont structurés avec différentes hauteurs pour piéger différentes particules (18a à 18c) à différents emplacements de niveau sur la base de la taille des particules ; lesdits niveaux ou étapes (16a à 16c) étant soutenus, de préférence, avec des piliers pour éviter l'effondrement de la structure ; et
e) une zone de sortie (20) pour la solution d'échantillon de rebut.

2. Dispositif LsP (2) selon la revendication 1, dans lequel la zone d'entrée (4) est conçue comme zone d'injection de gouttes de sang.

3. Dispositif LsP (2) selon la revendication 1, dans lequel les réseaux DLD sont réalisés pour une période N = 1, 2, 3, 10, 20 avec un espacement entre piliers de 1 µm ou pour une période N = 1, 10, 12, 18, 25 avec un espacement entre piliers de 500 nm.

4. Procédé de séparation des particules selon la taille, de piégeage et de manipulation de micro / nanoparticules dans un flux d'une solution d'échantillon pour la détection moléculaire ; comprenant les étapes de :
a) fourniture d'une solution d'échantillon à examiner ;
b) acheminement - essentiellement par force capillaire - de la solution d'échantillon à travers un dispositif LsP(2) selon l'une quelconque des revendications précédentes 1 à 3.

5. Procédé selon la revendication 4, dans lequel avant d'introduire la solution d'échantillon, une solution comprenant des nanoparticules fonctionnalisées est acheminée à travers le dispositif LsP (2) ; ladite fonctionnalisation met en évidence les biomolécules destinées à être piégées dans la zone de piégeage et de détection de nanoparticules fonctionnalisées (12) dudit dispositif LsP (2).

6. Procédé selon la revendication 4, dans lequel une solution comprenant des nanoparticules fonctionnalisées est ajoutée à la solution d'échantillon avant que la solution d'échantillon soit acheminée à travers le dispositif LsP (2) ; ladite fonctionnalisation met en évidence les biomolécules destinées à être piégées dans la zone de piégeage et de détection des nanoparticules fonctionnalisées (12) dudit dispositif LsP (2).

7. Procédé selon la revendication 5 ou 6, dans lequel une charge de biomolécules piégée dans la zone de piégeage et de détection de nanoparticules fonctionnalisées (12) dudit dispositif LsP (2) est étudiée en utilisant une détection optique pour acquérir un signal de SERS (spectroscopie Raman amplifiée en surface) du complexe anticorps-antigène ou un signal fluorescent provenant de l'ensemble moléculaire fixé à la surface de ces particules.
